# FASCICULE DE BREVET EUROPEEN

(11) **EP 1 567 200 B1**
(45) Date de publication et mention de la délivrance du brevet: **30.08.2006**
(21) Numéro de dépôt: 03778200.0
(22) Date de dépôt: 01.12.2003
(51) Int. Cl.: A61L 2/14

(54) **PROCEDE DE STERILISATION PAR PLASMA D'OBJETS DE NATURE DIELECTRIQUE ET COMPORTANT UNE PARTIE CREUSE**
VERFAHREN ZUR PLASMASTERILISATION VON DIELEKTRISCHEN GEGENSTÄNDEN MIT HOHLEN TEILEN
METHOD FOR THE PLASMA STERILISATION OF DIELECTRIC OBJECTS COMPRISING A HOLLOW PART

(30) Priorité: 02.12.2002 CA 2412997
(43) Date de publication de la demande: 31.08.2005
(73) Titulaire: Valorisation-Recherche, Limited Partnership, Montreal, QC H3X 2H9 (CA)
(72) Inventeur: POLLAK, Jérôme, Montréal, Québec H3T 1J6 (CA); MOISAN, Michel, Outremont, Québec H2V 2Z1 (CA); SAOUDI, Bachir, Montréal, Québec H4A 2K3 (CA); ZAKRZEWSKI, Zenon, PL-80-461 Gdansk (PL)
(74) Mandataire: Dronne, Guy
(86) Numéro de dépôt international: PCT/CA2003/001867
(87) Numéro de publication internationale: WO 2004/050128

(56) Documents cités:
- EP-A- 1 084 713
- WO-A-00/72889
- US-A- 4 643 876
- US-A1- 2002 172 780

## Description

### DOMAINE DE L'INVENTION

La présente invention est relative à un nouveau procédé permettant la stérilisation d'objets comportant des parties creuses, de nature diélectrique, contaminés par des micro-organismes et également par des agents transmissibles non conventionnels comme le prion pathogène. Lors de la mise en oeuvre de ce procédé, les objets contaminés, ainsi qu'éventuellement l'emballage correspondant, sont directement soumis alternativement à la post-décharge d'un plasma et à un champ électromagnétique d'une intensité suffisante pour créer un plasma à l'intérieur des parties creuses.

### ART ANTÉRIEUR

Traditionnellement, la stérilisation des instruments chirurgicaux est conduite en milieu hospitalier par imprégnation à l'aide de fluides ayant une action anti-bactérienne et/ou antivirale, tels que le glutaraldéhyde et le peroxyde d'hydrogène.

D'autres méthodes communément employées pour la stérilisation d'objets contaminés reposent sur le traitement thermique des objets à haute température. Ces procédés présentent néanmoins l'inconvénient d'endommager significativement un grand nombre de polymères qui constituent, en tout ou en partie, les instruments médicaux et dentaires.

Enfin des méthodes de stérilisation associant un traitement thermique et un traitement par liquide désinfectant ont été proposées. Cependant, outre leur plus grande complexité opérationnelle, ces méthodes présentent l'inconvénient d'être associés à des durées opérationnelles élevées.

Récemment, de nouvelles techniques de stérilisation par plasma ont été proposées. Les demandes de brevets EP-A-1181062 et CA-A-2.395.659 décrivent ces procédés ainsi que les dispositifs permettant la stérilisation d'objets médicaux en recourant à la post-décharge d'un plasma, par exemple d'argon ou d'un mélange de N₂-O₂. Ces procédés et dispositifs se sont révèles particulièrement adaptés pour la stérilisation des objets à usage médical, tels que des scalpels ou des pinces chirurgicales, dépourvus de cavités, d'un diamètre inférieur à quelques millimètres et de longueur dépassant le mètre.

Ces procédés sont, en effet, d'une application limitée en ce qui concerne la désinfection d'objets comportant des cavités profondes tels que des conduits. Ceci vient de la faible conductance hydrodynamique de tels objets qui rend difficile d'y faire circuler un gaz, à grande vitesse, condition pourtant requise pour utiliser les espèces actives (émetteurs d'UV et radicaux), à durée de vie limitée, créées dans une source de plasma extérieure au conduit (procédé dit de post-décharge), afin que celles-ci parviennent à inactiver les micro-organismes sur toute la surface interne d'un tel conduit. Par ailleurs, une limitation particulière à la stérilisation par plasma (que ce soit par exposition dans la décharge elle-même ou dans sa post-décharge) réside dans le traitement d'objets préemballés, qui est une façon de faire commune à toutes les techniques de stérilisation actuellement existantes. En effet, le passage des espèces actives d'un plasma ou de sa post-décharge à travers l'emballage en réduit d'une manière importante le flux atteignant les surfaces de l'objet à stériliser.

Le brevet US-A-5.393.490 décrit un procédé de stérilisation de la surface d'objets contaminés par exposition aux espèces électriquement neutres d'une décharge électrique, tout en maintenant le volume sans luminescence et substantiellement libre de champ en interposant une barrière entre les objets contaminés et la décharge. La barrière (une grille métallique) est transparente aux espèces neutres et opaque aux espèces chargées émanant de la décharge. Dans ce cas, le flux qui atteint les objets contamines n'est plus un plasma mais une post-décharge. La température y est maintenue basse par mise en oeuvre de systèmes de refroidissement et par le choix du (ou des) gaz utilisé(s) pour générer la post-décharge. La nature de ces gaz, notamment celle des gaz fluorés, est souvent telle qu'une dégradation accélérée des objets traités est à prévoir.

Le brevet US-A-5.302.343 décrit une méthode de stérilisation similaire pour la décontamination de la surface des objets contaminés en les exposant à des espèces stérilisantes neutres.

Le brevet US-A-6.589.481 décrit l'utilisation d'un système de pompe pour la stérilisation du lumen en présence de peroxyde.

Le brevet US-A-3.948.601 décrit un traitement entièrement post-décharge de l'intérieur et de l'extérieur d'un objet contaminé.

La demande internationale WO02070025 décrit un procédé de stérilisation par plasma dans lequel les objets contaminés sont placés dans une chambre à pression atmosphérique.

Il existait donc un besoin pour une méthode de stérilisation dépourvue d'au moins une des limitations des méthodes de l'art antérieur et permettant notamment une stérilisation sécuritaire, rapide, économique et/ou hautement performante en.ce qui concerne sa finalité.

### BRÈVE DESCRIPTION DES FIGURES

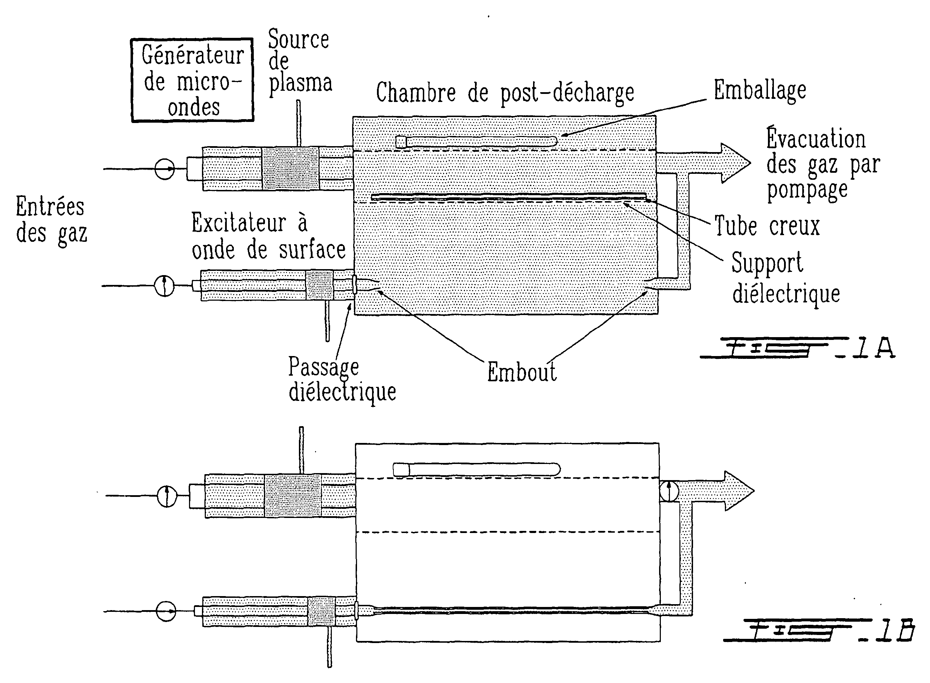

**La Figure 1** représente un dispositif de stérilisation par plasma selon un mode de réalisation de la présente invention. La stérilisation s'effectue en deux étapes dont l'ordre peut varier : L'étape 1 correspond à la stérilisation de l'extérieur du tube creux alors que l'étape 2 correspond à la stérilisation de l'intérieur du tube creux dans lequel circule un gaz; dans ce cas, l'enceinte contenant le tube creux est mise sous pression réduite : aucun gaz n'y circule. Les parties colorées en gris sont remplies d'un gaz susceptible de produire un plasma et les parties non colorées son sous vide. Le débit des gaz utilisés est contrôlé à l'aide de débitmètres.

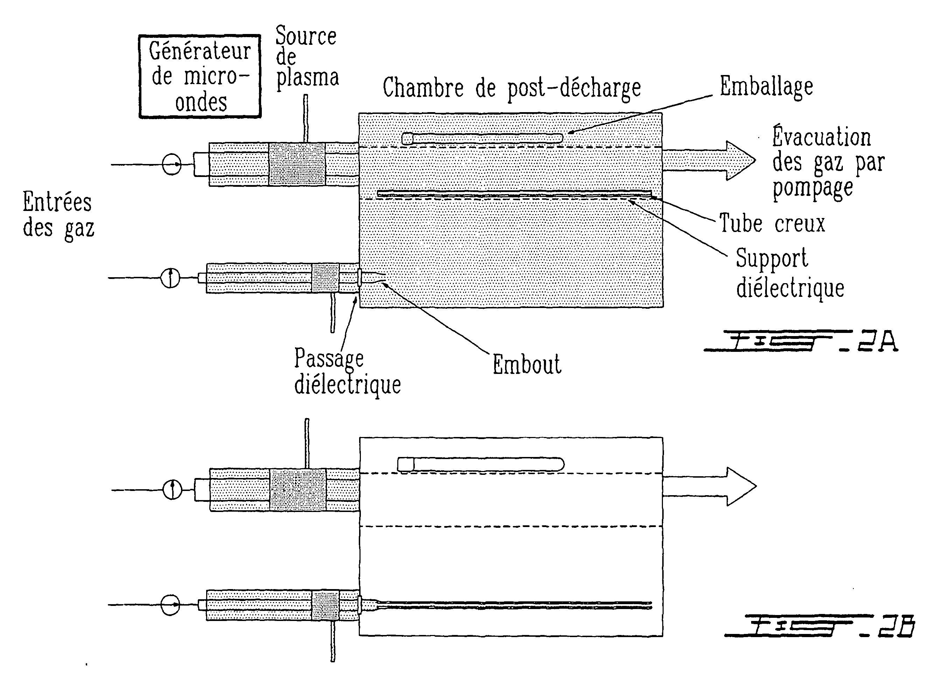

**La Figure 2** représente une autre variante de réalisation de la présente invention. Dans l'étape 2 qui correspond dans ce cas à la stérilisation de l'intérieur du tube creux, l'extrémité du tube creux à stériliser, opposée à l'excitateur d'ondes de surface, est libre (sans embout). L'évacuation des gaz du plasma se fait par le même orifice que celui utilisé lors du fonctionnement en post-décharge. Les parties colorées en gris sont remplies d'un gaz susceptible de produire un plasma et les parties non colorées sont sous vide. Le débit des gaz utilisés est contrôlé à l'aide d'un débitmètre massique.

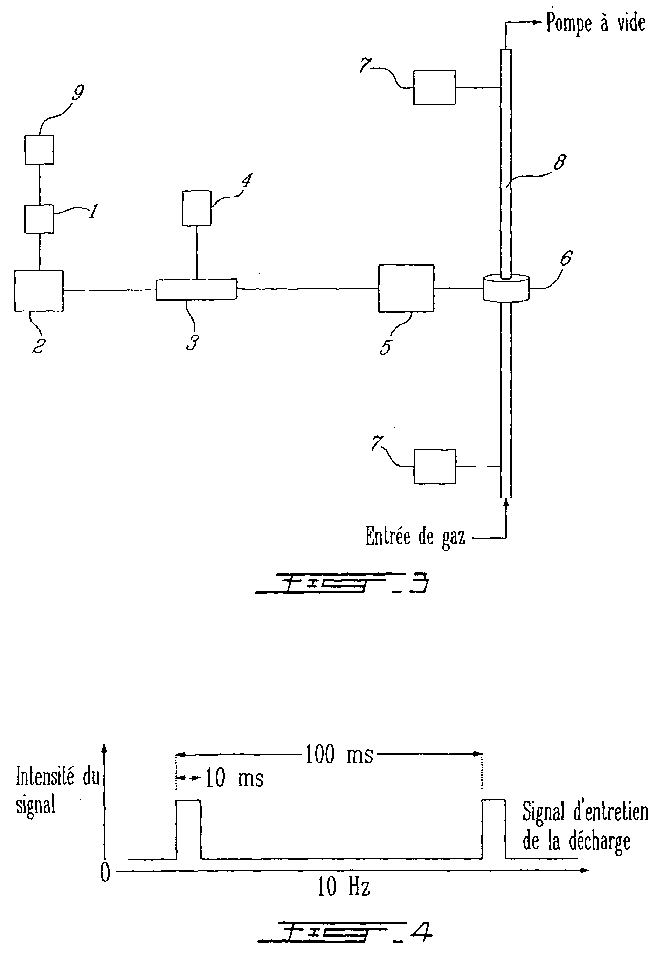

**La Figure 3** représente un montage, selon un mode de réalisation de l'invention, de l'utilisation de la propagation d'une onde de surface pour produire un plasma à l'intérieur d'un tube creux en matériau diélectrique sans que celui-ci ne soit endommagé par la chaleur, en contrôlant la température du gaz en agissant sur le temps pendant lequel l'onde électromagnétique est générée dans la décharge gazeuse.

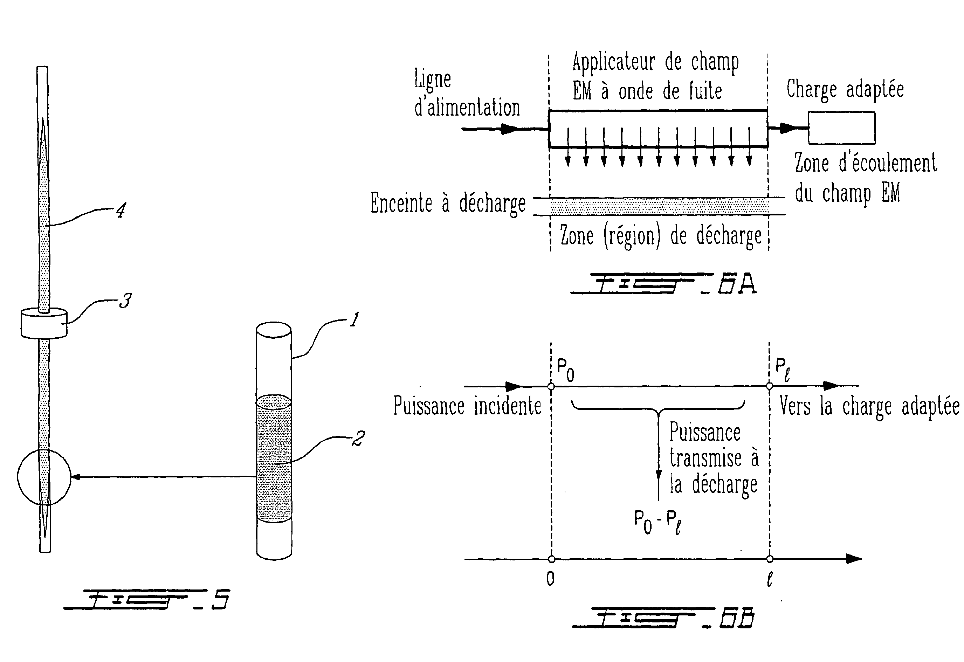

**La Figure 4** représente l'impulsion en créneau pilotant l'oscillateur alimentant l'amplificateur qui produit la puissance électromagnétique requise pour créer la décharge électrique.

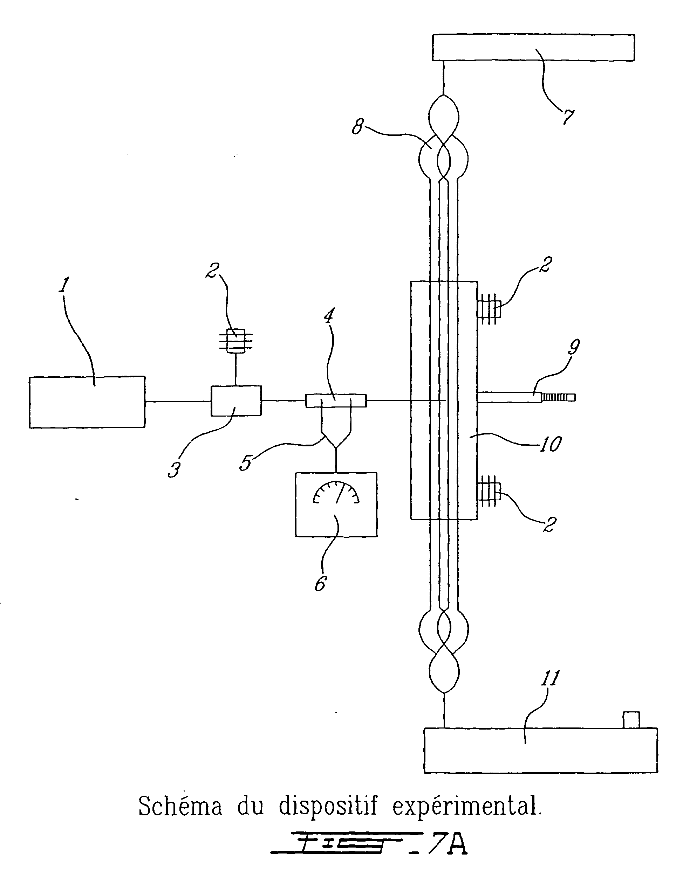

**La Figure 5** montre une façon de vérifier facilement l'activité stérilisante d'un dispositif selon l'invention en insérant une section de tube en Téflon^{™}, mince, à l'intérieur du tube à décharge. Cette section a été préalablement contaminée avec 50 µlitre d'une suspension contenant 10⁶ spores de *B. subtilis.*

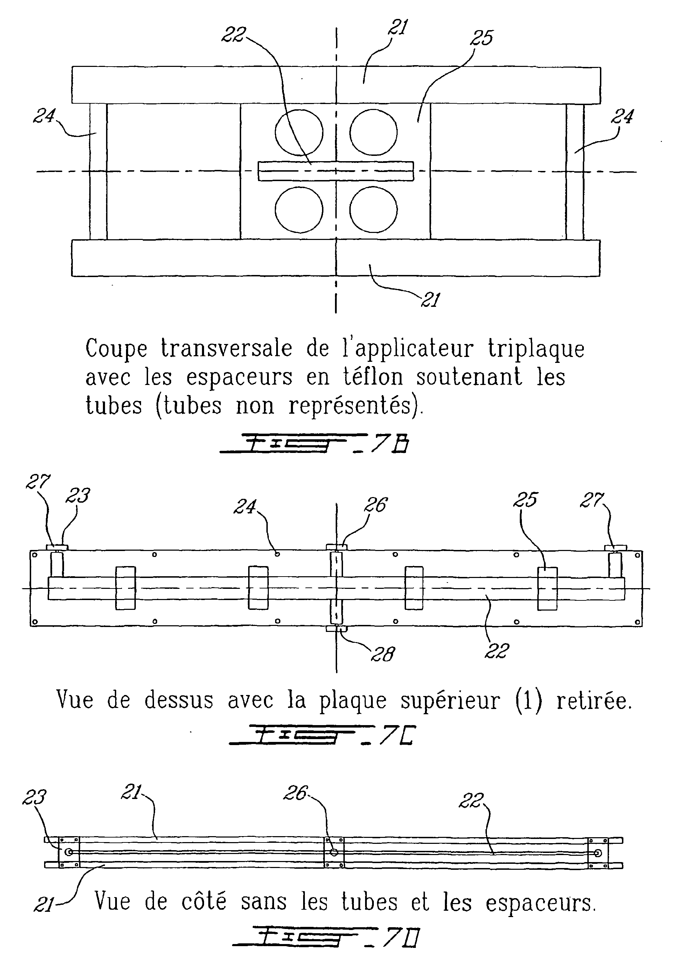

**La Figure 6A** représente un schéma de principe d'utilisation d'un applicateur de géométrie linéaire pour alimenter un tube à décharge qui lui est co-linéaire et tel que décrit dans la publication de G. Sauvé, M. Moisan, Z. Zakrzewski, C. B. Bishop, IEEE Transactions on Antennas and Propagation, 43, 248-256 (1995).
**La Figure 6B** représente la distribution des puissances liées à l'utilisation de l'applicateur de la Figure 6A.
**La Figure 7A** montre un type particulier d'applicateur linéaire, système dit tri-plaques, conforme au schéma de principe de la Figure 6A mais particulièrement adapté à la stérilisation simultanée de plusieurs tubes creux. Ce dispositif fonctionne, dans cet exemple, à la fréquence de 915 MHz. La Figure représente un dispositif erxpérimenatl selon l'invention mettant en oeuvre un applicateur tri-plaque. L'élément (1) représente un générateur HF, (2) une charge adaptée, (3) un circulateur, (4) une ligne bi-directionnelle, (5) une bascule (commutateur, (6) un bolomètre, (7) une bouteille de gaz, (8) un système de division du flux de gaz, (9) un piston court-circuit pour accord d'impédance, (10) un applicateur triplaque et (11) un système de pompage.
**La Figure 7B** représente une coupe transversale de l'applicateur triplaque représenté sur la Figure 7A par la numérotation 10 avec des espaceurs en Téflon™ soutenant des tubes non représentés. Avec (21) qui représente une plaque métallique, (22) une bande conductrice centrale, (23) un connecteur N, (24) un espaceur en métal, (25) un espaceur en Téflon™, (26) une arrivée de puissance, (27) un branchement possible de charges adaptées et (28) un branchement possible d'un piston court-circuit pour accord d'impédance.
**La Figure 7C** représente une vue de dessus du dispositif de la Figure 7A et de la Figure 7B, avec la plaque supérieure retirée du système triplaque.
**La Figure 7D** représente une vue de côté sans les tubes et sans les espaceurs.

### RÉSUMÉ DE L'INVENTION

La présente invention met à disposition un procédé pour la stérilisation d'objet contaminés à parties creuses. Ce procédé comporte au moins une étape dans laquelle la présence d'un champ électromagnétique, soit généré intrinsèquement par propagation d'une onde de surface, soit appliqué de l'extérieur, génère un plasma dans les parties creuses de l'objet contaminé. Ce traitement peut être combiné avec le traitement stérilisant d'une post décharge d'un plasma. La stérilisation des objets contaminés ainsi traités présente notamment l'avantage d'être réalisée de façon très performante et sans dégradation des objets traités.

### DÉFINITION GÉNÉRALE DE LINVENTION

**Un premier objet** de la présente invention est relatif à un procédé permettant de stériliser un objet contaminé de nature diélectrique et qui comporte au moins une partie creuse. Ce procédé comporte au moins une étape dans laquelle au moins un champ électromagnétique, d'une intensité suffisante pour créer un plasma à l'intérieur d'un gaz ou d'un mélange de gaz introduit dans la ou les partie(s) creuses de l'objet contaminé, est directement appliqué depuis l'extérieur de l'objet contaminé dans la ou les partie(s) creuse(s) ou généré intrinsèquement à l'intérieur de la ou des partie(s) creuse(s) de l'objet contaminé. Ce procédé permet également de traiter simultanément plusieurs objets contaminés.

À titre d'exemple d'un champ électromagnétique qui est directement appliqué de l'extérieur on peut citer le cas des applicateurs linéaires.

À titre d'exemple d'un champ électromagnétique généré intrinsèquement à l'intérieur des surfaces creuses, on peut mentionner celui lié aux ondes de surface, qui se propagent grâce au plasma qu'elles créent le long de l'objet dont on veut stériliser l'intérieur.

Dans le cadre de la présente invention on appelle onde de surface une onde dont le support de propagation est un (ou plusieurs) milieu(x) diélectrique(s). Lors de la mise en oeuvre du procédé de la présente invention, deux diélectriques sont impliqués, celui de l'objet creux et celui constitué par le plasma lui-même. En effet, ce dernier peut être effectivement considéré comme un milieu diélectrique.

Dans le cadre de la présente invention on appelle objet de nature diélectrique un matériau globalement de conductivité électrique très faible soit transparent aux champs électromagnétiques (EM) et de façon à ce qu'il ne chauffe pas de façon significative sous son action.

Parmi les micro-organismes indésirables susceptibles d'être ainsi détruits, on peut mentionner les virus, spores, bactéries, champignons, moisissures et prions.

Lors de la mise en oeuvre de ce procédé, les objets contaminés, ainsi qu'éventuellement l'emballage correspondant, sont directement soumis alternativement à la post-décharge d'un plasma et à un champ électromagnétique d'une intensité suffisante pour créer un plasma à l'intérieur des parties creuses.

Selon un mode préférentiel de l'invention, le champ électromagnétique est celui d'une onde de surface qui se propage à la fois sur l'intérieur et l'extérieur du tube creux de l'objet contaminé et ce grâce au plasma qu'elle crée à l'intérieur du tube creux.

Selon un autre mode avantageux, le champ électromagnétique est appliqué, à l'intérieur de la ou des partie(s) creuse(s) de l'objet contaminé, depuis l'extérieur de l'objet contaminé. Ceci est réalisé par exemple à l'aide d'un applicateur linéaire.

De préférence, les parois et la masse en volume de la ou des partie(s) creuse(s) de l'objet contaminé sont essentiellement constituées d'un matériau diélectrique.

Avantageusement, les principales étapes du procédé de l'invention peuvent être résumées comme suit:
- a) soumission directe de la ou les partie(s) creuse(s) de l'objet contaminé à une stérilisation par un plasma généré par au moins un champ électromagnétique, de préférence par celui d'une onde électromagnétique de surface se propageant sur les surfaces interne et externe de la ou des partie(s) creuse(s); dans ce cas, l'extérieur du tube creux est préférentiellement sous vide dit primaire c'est à dire d'une valeur variant de 1.33 à 6.67 Pa (10 à 50 m Torr); et
- b) soumission de l'objet contaminé, de préférence essentiellement son extérieur, à une stérilisation par une post-décharge créée par un générateur de plasma.

La présente invention concerne également un procédé- de stérilisation d'un objet contaminé de nature diélectrique et comportant au moins une partie creuse. Ce procédé est caractérisé en ce que :
a) on génère un plasma à partir d'un gaz ou d'un mélange de gaz et d'un champ électromagnétique ;
b) on traite l'extérieur de l'objet par le biais d'une post-décharge du plasma; et
c) on traite la partie creuse de l'objet par le biais d'une décharge du plasma, la décharge étant générée à l'intérieur de la partie creuse,
l'étape (c) étant effectuée avant ou après l'étape (b).

La présente invention concerne aussi un procédé de stérilisation d'un objet contaminé de nature diélectrique et comportant une partie creuse, ledit procédé étant caractérisé en ce que :
a) on génère un premier plasma à partir d'un gaz ou d'un mélange de gaz et d'un champ électromagnétique ;
b) on traite l'extérieur de l'objet par le biais d'une post-décharge du plasma;
c) on génère un deuxième plasma à partir d'un gaz ou d'un mélange de gaz et d'un autre champ électromagnétique ;
d) on traite la partie creuse de l'objet par le biais d'une décharge du deuxième plasma, la décharge étant générée à l'intérieur de la partie creuse.

L'étape (c) est effectuée avant ou après l'étape (a) et/ou l'étape (b), et l'étape (d) est effectuée après l'étape (c).

Un plasma est un milieu, généralement gazeux, dans lequel on trouve des espèces chargées électriquement, comme les électrons et les ions, mais aussi des espèces électriquement neutres. On peut obtenir un plasma en appliquant au gaz un champ électrique suffisamment intense pour que se produise une décharge électrique dans ce gaz. Les espèces présentes dans la post-décharge y ont été amenées par un écoulement gazeux à partir de la zone où le plasma est créé. La recombinaison des ions et des électrons pour reformer des particules neutres est extrêmement rapide, plus rapide que le temps nécessaire pour les espèces d'arriver dans la zone de post-décharge. Il ne reste essentiellement plus alors dans cette dernière que des espèces neutres, dans leur état fondamental ou excité, y compris dans leur état ionisé.

Le procédé de l'invention peut être appliqué non seulement à la stérilisation d'un objet contaminé comportant au moins une partie creuse mais également à son emballage, ledit procédé comporte alors les étapes suivantes qui consistent dans :
a) la soumission de la ou les partie(s) creuse(s) desdits objets à une stérilisation par un plasma directement généré dans la ou les parties creuses par une onde électromagnétique de surface s'étendant sur les surfaces interne et externe de la ou des partie(s) creuse(s);
b) la soumission de l'objet contaminé à une stérilisation effectuée par une post-décharge;
c) la soumission de l'emballage à une stérilisation effectuée par une post-décharge; et
d) l'introduction dans un milieu stérile de l'objet creux stérilisé dans l'emballage stérilisé.

Les étapes a), b) et c) peuvent être réalisées dans un ordre indifférent, l'une après l'autre ou simultanément.

La stérilisation conjointe d'un objet contaminé et de son emballage d'une taille et d'une forme adaptée se fait de préférence dans une enceinte de stérilisation équipée d'un dispositif permettant la production d'une post-décharge par plasma ainsi que d'un dispositif permettant la production d'une onde électromagnétique de surface dans les parties creuses de l'objet contaminé. L'air initialement présent dans l'enceinte est avantageusement évacué jusqu'à une pression réduite qui est de préférence comprise entre 1.33 et 6.67 Pa (10 et 50 m Torr).

Cette étape est suivie de la stérilisation de l'objet contaminé par soumission à une stérilisation par post-décharge. Puis d'une étape dans laquelle l'emballage est soumis à une stérilisation par post-décharge et ensuite d'une étape, dans laquelle la ou les partie(s) interne(s) de l'objet creux contaminé sont soumises à stérilisation à l'aide du plasma généré par l'onde électromagnétique de surface qui s'étend préférentiellement sur toute la longueur dudit conduit. Ces différentes étapes peuvent être réalisées dans un ordre indifférent.

Le procédé de l'invention donne des résultats particulièrement avantageux lorsque l'objet contaminé est un conduit creux de forme cylindrique. Les dimensions de ce cylindre sont avantageusement choisies de façon à ce que le rapport obtenu en divisant la longueur du cylindre constitutif de l'objet contaminé par le diamètre du cylindre est compris entre 5.10³ et 0,3.10³.

La stérilisation par post décharge est réalisée selon l'un des procédés décrits dans la demande européenne EP-A-1181062 plus particulièrement dans les revendications correspondantes ainsi que dans la demande internationale WO 2004011039 plus particulièrement dans les revendications correspondantes.

Ce type de décharge correspond avantageusement à un plasma comprenant des espèces stérilisantes générées *in situ* par la soumission d'un flux gazeux comprenant entre 0.5 et 20% d'oxygène atomique, à un champ électrique suffisamment intense pour générer le plasma. Les espèces stérilisantes permettent la destruction de micro-organismes. Il est à noter que le gaz ne présente aucune propriété biocide avant son passage dans le champ électrique, et que le pourcentage d'oxygène atomique dans le flux gazeux est ajusté de façon à obtenir un rayonnement UV d'intensité maximale. De préférence, l'exposition a lieu dans la zone de post-décharge ou dans la zone d'excitation du plasma.

Le champ électrique de la décharge est alors avantageusement généré par une décharge micro-ondes et les espèces stérilisantes comprennent notamment des photons, des radicaux, des atomes et/ou des molécules.

Avantageusement, le flux gazeux utilisé comprend, en plus de l'oxygène atomique, de l'azote, du néon, de l'argon, du krypton, du xénon, de l'hélium, de l'oxygène, du monoxyde de carbone, du dioxyde de carbone, des oxydes d'azote, de l'air, et leurs mélanges.

Selon une autre variante, le flux gazeux comprend, en plus de l'oxygène atomique, de l'azote, l'argon et leurs mélanges, de préférence la proportion d'oxygène dans le flux gazeux varie entre 2% et 5%.

La température dans la post-décharge est de préférence inférieure ou égale à 50°Celsius. Ce traitement de post décharge est réalisé indifféremment de façon isolée ou répétitive dans un procédé séquentiel à plusieurs étapes.

Les étapes de la post-décharge envisageables comprennent notamment un gaz pulsé dans un champ électrique ou électromagnétique continu, un champ pulsé dans un gaz en flux continu, un gaz pulsé dans un champ pulsé de façon synchrone, un changement de gaz, ou un mélange de ces étapes.

Un dispositif de stérilisation permettant de réaliser une telle stérilisation par post-décharge est également décrit dans la demande de brevet EP-A-1181062 comme comprenant une source de plasma couplée à une chambre de stérilisation par un tube à décharge dans lequel est injecté un gaz ou un mélange de gaz générant éventuellement le plasma, la chambre comprenant un objet à stériliser, et une pompe à vide pour entraîner les gaz dans la chambre et y maintenir une pression réduite. La source de plasma comprend un applicateur de champ EM tel qu'un surfatron ou un surfaguide. La chambre de stérilisation est fabriquée entièrement ou partiellement avec du Pyrex^{™} ou en aluminium, par exemple.

Un procédé de stérilisation par post-décharge d'objets contaminés encore plus performant est décrit dans la demande WO-A-2004011039 précitée.

Ce procédé est mis en oeuvre dans une enceinte de stérilisation équipée d'au moins un conduit de décharge, le ou les conduit(s) de décharge arrive(nt) dans l'enceinte de stérilisation et est (sont) alimenté(s) par un flux d'alimentation liquide ou gazeux, les objets contaminés sont soumis dans l'enceinte de stérilisation à l'action d'espèces stérilisantes présentes dans une zone de post-décharge ou dans une zone d'excitation d'un plasma généré, au niveau du ou des conduit(s) de décharge, par soumission du flux gazeux d'alimentation à un champ électrique et le rapport R = (SCD)/(SCS), dans lequel (SCD) représente la section du conduit de décharge au contact de l'enceinte de stérilisation ou la somme des sections des conduit(s) de décharge et (SCS) la section de la chambre de stérilisation (SCS), vérifie la relation 0,05 < R < 0,70.

De préférence les caractéristiques structurelles de l'enceinte de stérilisation sont choisies de façon que R vérifie la relation 0,09 ≤ R ≤ 0,60, plus préférentiellement encore 0,15 ≤ R ≤ 0,5. Selon un mode particulièrement intéressant 0,2 ≤ R ≤ 0,40, de préférence R est voisin de 0,25.

Le champ électrique qui génère le plasma est avantageusement un champ à haute fréquence dont la fréquence est habituellement comprise entre 10 Mégahertz et 3 Gigahertz, et qui varie de 100 à 2.450 MHz. selon un mode plus avantageux encore la fréquence est comprise entre 200 et 915 MHZ.

Le flux gazeux d'alimentation peut être ajusté par réglage du débit et/ou de la pression du gaz dans l'enceinte, de façon à obtenir un rayonnement Ultra Violet (UV) d'intensité maximale. Il est avantageusement choisi de façon à ce que son débit soit compris entre 50 et 3.000 cm³ par minute.

La pression générée à l'intérieur de l'enceinte de stérilisation est comprise entre 13.3 et 1330 Pa (0,1 et 10 Torr).

Le flux gazeux d'alimentation comprend avantageusement de l'argon et la pression générée à l'intérieur de l'enceinte de stérilisation est comprise entre 13.3 et 533 Pa (0,1 et 4 Torr).

Selon une autre variante, le flux gazeux comprend de l'azote et de l'oxygène moléculaire et la pression générée à l'intérieur de l'enceinte de stérilisation est comprise entre 133 et 1070 Pa (1 et 8 Torr).

Le flux gazeux d'alimentation comporte au moins un composant choisi dans le groupe constitué par l'oxygène moléculaire, l'azote, le néon, l'argon, le krypton, le xénon, l'hélium, l'oxygène, le monoxyde de carbone, le dioxyde de carbone, les gaz de formule NOₓ ,avec x représentant un nombre entier sélectionné dans le groupe constitué par 1, 2 ou 3, l'air, et les mélanges d'au moins deux ou de plusieurs de ces derniers.

Selon un mode avantageux, le flux gazeux d'alimentation comporte de l'oxygène moléculaire. Préférentiellement, le flux gazeux comporte au moins 0,10 % d'oxygène moléculaire, plus avantageusement encore au moins 0,04 % d'oxygène moléculaire.

Selon une autre variante intéressante le flux gazeux d'alimentation comporte en plus de l'oxygène moléculaire, au moins deux autres gaz.

Ainsi, en plus de l'oxygène moléculaire, au moins trois autres gaz qui peuvent être par exemple de l'azote, de l'argon et de l'hélium ou bien de l'azote, de l'argon et du dioxyde d'azote ou bien encore de l'oxygène moléculaire de l'azote, du xénon ou du krypton peuvent être présents dans le flux gazeux d'alimentation.

Le débit du gaz d'alimentation est compris entre 10 et 5.000 cm³ standard par minute, préférentiellement ce débit est compris entre 50 et 3.000 cm³ par minute.

Lorsque le flux gazeux est constitué de NO₂ d'azote, ou d'un mélange oxygène - azote et la pression générée à l'intérieur de l'enceinte de stérilisation est comprise entre 267 et 1070 Pa (2 et 8 Torr).

À titre illustratif le flux gazeux présente la composition suivante :
- de 0,04 à 30 % d'O₂;
- de 0,05 à 99,91 % d'azote; et
- de 0,05 à 99,91 % d'argon.

Ou bien le flux gazeux présente la composition suivante :
- de 0,04 à 30 % d'O₂;
- de 0,05 à 99,91 % d'azote ; et
- de 0,05 à 99,91 % de krypton.

Ou bien encore, le flux gazeux présente la composition suivante :
- de 0,04 à 99,90 % d'O₂ ;
- de 0,05 à 99,91 % d'azote; et
- de 0,05 à 99,91 % de xénon; ou
- de 0,05 à 99,91 % de néon.

Selon un autre mode avantageux de réalisation, le flux gazeux présente la composition suivante:
- de 0,04 à 98,5 % d'O₂;
- de 0,05 à 99,6 % d'azote;
- de 0,05 à 99,6 % de xénon; et
- de 0,05 à 99,6 % de néon.

De préférence, le flux gazeux comporte de 0,1 à 10 % d'O₂ plus préférentiellement encore comporte de 0,2 à 5 % d'O₂.

Parmi les espèces stérilisantes ainsi produites dans la post-décharge on peut mentionner les photons, les radicaux, les atomes et/ou les molécules. La population de photons et/ou de radicaux est importante, et peut même être majoritaire.

Selon une autre variante intéressante de l'étape de stérilisation par post-décharge, on expose les objets contaminés, dans une enceinte de stérilisation, à un plasma généré dans au moins un conduit de décharge arrivant dans ladite enceinte, à partir d'un flux gazeux de N₂, ledit plasma comprenant des espèces stérilisantes générées lors de la soumission dudit flux gazeux à un champ électrique suffisamment intense pour générer le plasma. Ce procédé comprenant l'exposition des objets contaminés aux espèces stérilisantes, cette exposition a lieu dans une zone de post-décharge ou dans une zone d'excitation du plasma et il est caractérisé en ce que :
- le pourcentage d'oxygène moléculaire dans le flux de N₂ gazeux est ajusté à une teneur x, en oxygène moléculaire, telle que 0 < x < 0,5 (x variant préférentiellement de 0,1 à 0,4 %), de préférence par réglage du débit et/ou par réglage de la pression du gaz dans l'enceinte, de façon à obtenir un rayonnement UV d'intensité maximale;
- l'oxygène moléculaire est transformé au moins partiellement en oxygène atomique; et
- la section du conduit de décharge à son entrée dans la chambre de stérilisation (SCD) et celle de la chambre de stérilisation (SCS) vérifient la relation 0,05 < (SCD)/(SCS) < 0,7,
la section (SCD) représentant à nouveau la section du conduit de décharge au contact de la chambre de stérilisation et qui est perpendiculaire à la direction du flux gazeux alimentant le conduit de décharge et la section (SCS) représentant la section de la chambre au contact du conduit de décharge et qui est perpendiculaire au courant de plasma.

Comme variante pour la production d'une post-décharge adaptée dans le cadre de la mise en oeuvre du présent procédé de stérilisation selon l'invention on peut mentionner l'exposition des objets contaminés à un plasma généré dans au moins un conduit de décharge arrivant dans une enceinte de stérilisation, et ce à partir d'un flux gazeux comportant au moins un des gaz du groupe constitué par l'oxygène et les gaz rares comme l'hélium, le néon, l'argon, le krypton et le xénon. Ledit plasma comprenant des espèces stérilisantes générées lors de la soumission dudit flux gazeux à un champ électrique suffisamment intense pour générer le plasma, ledit procédé est caractérisé en ce que :
- le flux gazeux est ajusté, de préférence par réglage du débit et/ou par réglage de la pression du gaz dans l'enceinte, de façon à obtenir un rayonnement UV d'intensité maximale; et
- la section du conduit de décharge à son entrée dans la chambre de stérilisation (SCD) et celle de la chambre de stérilisation (SCS) vérifient la relation 0,05 < (SCD)/(SCS) < 0,70.

La température dans l'enceinte de stérilisation est alors avantageusement de 60° Celsius ou moins, et de préférence cette température est d'environ 30° Celsius. La durée de soumission à la post-décharge est comprise entre 10 minutes et 4 heures.

Cette étape de traitement par une post-décharge peut être réalisée de façon isolée ou répétitive dans un processus séquentiel à plusieurs étapes, par exemple en mettant en oeuvre un gaz pulsé dans un champ électrique ou électromagnétique appliqué de façon continu, un champ électrique pulsé dans un gaz en flux continu, un gaz pulsé dans un champ électrique pulsé de façon synchrone, un changement de gaz; ou une combinaison de ces étapes.

Ce procédé est avantageusement mis en oeuvre dans un dispositif tel qu'illustré dans les Figures 1 à 13 de la demande internationale WO-A-2004011039. Il comporte une source de plasma couplée à une des parois de la chambre de stérilisation par au moins un tube à décharge dans lequel est injecté un gaz ou un mélange de gaz générant éventuellement le plasma, la chambre comprenant les objets à stériliser, et une pompe à vide pour entraîner les gaz dans la chambre et y maintenir une pression réduite. La source de plasma comprend un applicateur de champ électrique et le rapport R vérifie la relation 0,05 < R < 0,7. À titre d'exemple l'applicateur de champ électrique est de type surfatron ou de type surfaguide.

Dans le cadre de la présente invention, on utilise de préférence un mélange N₂-O₂ pour produire la post-décharge, ce qui permet d'obtenir une excellente uniformité de la distribution des espèces actives dans l'enceinte de stérilisation.

Avantageusement, dans l'étape de soumission de l'objet contaminé à une post décharge, ledit objet est de type conduit creux comportant au moins deux extrémités libres, chacune des extrémités étant munie d'un embout et positionnée dans l'enceinte de stérilisation de façon à ce que le premier embout soit en contact avec un excitateur d'onde de surface et de façon que le deuxième embout soit relié à un système de pompage qui évacue les effluents de la décharge et, le cas échéant, des parties des micro-organismes passées sous forme gazeuse à l'extérieur de l'enceinte de stérilisation.

Ce procédé donne des résultats particulièrement intéressants dans la décontamination des endoscopes, des cathéters et de façon générale des conduits creux à axes parallèles disposés dans une enveloppe cylindrique ou oblate.

Les paramètres du cycle utile de puissance électromagnétique (ci-après appelé EM) générée au cours de la stérilisation, par exemple pendant la période de marche et celle d'arrêt, sont avantageusement ajustés pour éviter l'endommagement, par échauffement, de la paroi du canal du conduit.

Le cycle utile de puissance EM (de l'onde de surface) est quant à lui ajusté à une valeur comprise entre 1 et 100%.

Le temps de traitement de la ou des partie(s) creuse(s) par l'onde de surface électromagnétique est de préférence compris entre 45 et 120 minutes, de préférence le temps de traitement est d'environ 60 minutes et/ou la température dans le tube creux compris entre 30 et 60 degrés Celsius, de préférence compris entre 30 et 45 degrés Celsius.

La fréquence d'excitation du plasma créé dans la ou les partie(s) creuse(s) de l'objet contaminé est comprise entre 10 kHertz et 10 GigaHertz, de préférence cette fréquence est comprise entre 1 MHertz et 2500 MHertz.

L'emballage, disposé dans l'enceinte au même titre que le conduit, est maintenu ouvert à une extrémité et soumis à la stérilisation par la post-décharge ou par l'onde de surface. Le côté ouvert de l'emballage est alors préférentiellement orienté face à la source de plasma qui produit la post-décharge.

Une fois la stérilisation de l'objet contaminé terminée, l'objet est transféré, tout en maintenant une ambiance stérile, dans l'emballage présent dans l'enceinte.

Il est alors avantageux de procéder au scellement de l'emballage contenant l'objet à l'intérieur même de l'enceinte de stérilisation, par exemple par thermo-soudure.

L'objet contaminé et/ou l'emballage sont préférentiellement disposés sur des supports amovibles positionnés de façon indépendante dans l'enceinte de stérilisation. Ces supports sont éventuellement déplacés au cours dudit procédé en hauteur et latéralement, suivant les besoins des étapes de stérilisation et en fonction de la taille et de la forme des objets à décontaminer.

À titre illustratif on peut utiliser un générateur d'onde de surface de type SURFAGUIDE tel que celui commercialisé par la société Air Liquide, sous la référence UPAS.

Dans ces circonstances, les opérations de déplacement d'objets et/ou de support à l'intérieur de l'enceinte de stérilisation sont effectuées en ambiance stérile, à l'aide d'un bras articulé piloté extérieurement.

L'excitateur d'onde électromagnétique de surface est disposé à une extrémité du (des) conduit(s) à stériliser ou, de façon coaxiale au conduit, en tout point le long de cet objet.

L'applicateur de champ électromagnétique peut être est un applicateur de type capacitif.

L'applicateur de champ EM de type capacitif peut alors être constitué de plaques parallèles conductrices, les plaques parallèles peuvent être recouvertes d'un matériau diélectrique, disposées de part et d'autre de l'objet à décontaminer, ces plaques parallèles sont avantageusement alimentées par un générateur de puissance EM.

L'applicateur de champ électromagnétique est avantageusement constitué de spires qui génèrent dans le conduit diélectrique un champ électromagnétique, ce champ induit la formation d'un plasma dans les parties creuses de l'objet à stériliser.

Les faces externes du (des) conduits creux qui ont reçu les embouts creux (empêchant leur stérilisation) sont soumises à stérilisation en l'absence desdits embouts, dans la même étape que la partie extérieure de l'objet contaminé et/ou que son emballage.

Un deuxième objet de la présente demande est constitué par un dispositif de stérilisation comportant une chambre de stérilisation, ladite chambre est équipée d'une source de post-décharge et d'un générateur d'ondes de surface et/ou d'un applicateur de champ EM de forme linéaire, ces deux derniers étant susceptibles de générer un plasma dans la partie creuse d'un objet diélectrique.

La chambre de stérilisation de ce dispositif est alimentée par une post-décharge de plasma et équipée d'un générateur d'ondes de surface.. La chambre de stérilisation peut aussi comporter des moyens permettant de manipuler de façon stérile les objets qui y sont placés.

Le dispositif est muni d'un dispositif d'évacuation des gaz du plasma formé dans l'enceinte de stérilisation vers l'extérieur de ladite enceinte.

Un tel dispositif est illustré dans les Figures 1A et 1B ainsi que dans les Figure 2A et 2B.

La présente invention concerne également un dispositif permettant de stériliser un objet contaminé de nature diélectrique et ayant au moins une partie creuse. Le dispositif comprend :
- une enceinte de stérilisation destinée à recevoir l'objet ;
- une première source de plasma en communication avec l'enceinte, et destinée à générer un plasma servant à traiter l'extérieur de l'objet par le biais d'une post-dècharge ;
- une seconde source de plasma en communication avec l'enceinte, et destinée à générer un plasma servant à traiter la partie creuse de l'objet par le biais d'une décharge, la seconde source comprenant un embout dimensionné de telle sorte que lorsque ce dernier est en contact avec la partie creuse de l'objet, la décharge est produite à l'intérieur de la partie creuse; et
- une sortie en communication avec l'enceinte et permettant d'évacuer des gaz générés dans l'enceinte.

Un troisième objet de la présente invention est constitué par un procédé de stérilisation d'objets contaminés de nature diélectrique, comportant au moins une partie creuse, placés à l'intérieur d'un emballage scellé ou non scellé. Ce procédé comporte au moins une étape dans laquelle au moins un champ électromagnétique est généré à l'intérieur de la ou des partie(s) creuse(s) des objets contaminés et/ou de l'intérieur de l'emballage.

Plusieurs objets peuvent également être traités simultanément dans la même enceinte de stérilisation et par mise en oeuvre du procédé de l'invention.

Il existe un avantage certain à procéder en deux étapes pour effectuer la stérilisation des objets contaminés, notamment du fait qu'il est difficile, sinon impossible avec certains gaz, de créer efficacement une décharge, en même temps, à l'intérieur d'un tube et sur son extérieur, lorsque les deux milieux baignent dans le même gaz à la même pression.

Le procédé tel que décrit dans les brevets américains US-A-5.302.343 et US-A-6.589.481 de Jacob nécessite la mise en oeuvre d'une barrière filtrant les particules chargées et qui ne laisse passer que les particules neutres. Dans ces conditions de fonctionnement, les espèces neutres auront du mal à pénétrer à l'intérieur du tube creux suffisamment rapidement pour être encore actives. En effet, on ne peut faire circuler un gaz à grande vitesse (condition nécessaire pour que les espèces soient encore actives) dans un tube de très faible diamètre sans y créer un fort gradient de pression c'est-à-dire une pression très forte côté admission du gaz et faible du côté pompe. Ainsi, les conditions d'optimisation des UV ne sont respectées que sur une petite section linéaire de l'intérieur du tube creux.

Dans ces circonstances, le procédé selon l'invention est plus rapide donc plus efficace.

Le procédé selon la présente invention résout ce problème en utilisant un flux de gaz quasi stationnaire (statique) et en y créant le plasma qui sera le même tout au long du tube à décharge, assurant ainsi une même efficacité de stérilisation le long d'un tube creux comme un endoscope.

L'onde de surface utilisée appartient à la catégorie des ondes EM : voir à ce sujet la publication de Margot et M. Moisan, *Characteristics of surface-wave propagation in dissipative cylindrical plasma columns,* J. Plasma Physics, vol. 49 pp. 357-374 (1993).

Un applicateur linéaire tel que décrit dans Sauvé et al. (1995) IEEE Transactions on Plasma Science, vol. 43, pp. 248-256 permet lui aussi d'imposer un champ EM à l'intérieur des parties creuses, mais qui n'est pas engendré par une onde de surface, mais plutôt une onde de fuite (leaky wave en anglais).

Un autre avantage du présent procédé de stérilisation par plasma réside dans le fait que la température du gaz de la décharge (T_{g}) reste en fonctionnement suffisamment faible soit (cas de l'onde de surface) du fait de l'imposition d'un fonctionnement discontinu à l'onde EM (notion d'impulsion), mais également de par la diminution de la fréquence de cette onde, ou bien encore (cas de l'applicateur linéaire) en faisant en sorte que le flux de puissance EM s'écoulant de l'applicateur de champ soit faible (par exemple, en pratiquant des trous suffisamment petits dans le guide d'onde dans lequel circule l'onde EM venant du générateur micro-ondes).

Alors que les méthodes décrites dans US-A-5.302.343 et dans US-A-5.393.490 préconisent, pour atteindre ce but, soit de refroidir l'enveloppe extérieure du système (voir Figure 14), soit de jouer sur la nature du mélange de gaz. Dans le cadre de la présente demande, pour aboutir à une optimisation de l'intensité d'émission des UV, on joue sur la composition du mélange ou sur sa pression. On ne peut généralement pas obtenir l'optimisation des UV et de la réduction de la Tg en même temps.

### DESCRIPTION DE MODES PRÉFÉRENTIELS DE RÉALISATION DE L'INVENTION

Les modes préférentiels ci-après décrits sont donnés à titre purement illustratifs et ne sauraient être interprétés comme constituant une quelconque limitation de la présente invention.

Grâce au procédé de l'invention, on a pu de façon surprenante, réaliser avec succès la stérilisation de cylindres creux de petit diamètre (de préférence d'un diamètre compris entre 0.5 - 4 mm) et de grande longueur (de préférence d'une longueur comprise entre 1.0 - 2.5 m). De tels cylindres sont réputés pour être intérieurement très difficilement stérilisables par une post-décharge en flux.

La propagation d'une onde de surface à l'intérieur du conduit génère un plasma (décharge) dans la partie creuse du conduit diélectrique à décontaminer. Les conditions opératoires sont optimisées de telle sorte qu'il y ait stérilisation sans échauffement dommageable de la paroi interne du conduit.

Par ailleurs, la partie extérieure de ce conduit peut être stérilisée par une post-décharge de plasma, par mise en oeuvre des techniques faisant l'objet de la demande européenne portant le numéro EP-A-1181062 et de la demande internationale WO-A-2004011039.

Selon un mode avantageux de réalisation de la présente invention, il est proposé de stériliser les objets hors de tout emballage et de procéder, une fois seulement le cycle de stérilisation terminé, à leur emballage.

L'emballage requis, placé initialement dans la même enceinte que le produit à stériliser, est préalablement rendu stérile par post-décharge et ce, de préférence, en même temps que l'objet à emballer. L'objet est alors transféré, sous ambiance stérile de l'enceinte, dans l'emballage, par exemple, au moyen d'une pince articulée. L'emballage est ensuite clos de façon étanche, par exemple, par thermo-soudure.

Un nouveau stérilisateur adapté pour la mise en oeuvre du procédé selon l'invention fait appel à deux modes de stérilisation, à savoir une post-décharge de plasma et un plasma d'onde de surface agissant de façon séquentielle dans la même enceinte. Ce dispositif comprend donc deux sources de plasma (entretenues par exemple par des micro-ondes) avec leur applicateur de champ électrique (un lanceur d'ondes dans le cas d'un plasma d'ondes de surface). Ce système, constitué d'une chambre de stérilisation unique, est maintenu à pression de gaz réduite, en présence d'un flux de gaz.

### Utilisation d'un plasma d'ondes de surface

Lorsque le matériau du conduit est à base de polymères, donc en général un bon diélectrique, il est possible d'utiliser la nature diélectrique de sa structure pour en faire le milieu de propagation d'une onde de surface électromagnétique (EM) qui, en même temps, va créer et entretenir un plasma dans son intérieur. On produit ainsi un plasma à l'intérieur du canal sans avoir à y introduire un conducteur (une électrode), comme cela serait nécessaire si l'on voulait réaliser une décharge en courant continu (champ électrique d'intensité constante), par exemple.

L'entretien d'une colonne de plasma, à l'intérieur d'un conduit (tube) diélectrique (verre, silice fondue, par exemple), par une onde EM de surface guidée par ce tube est décrite dans la publication de M. Moisan et Z. Zakrzewski, intitulée "Plasma sources based on the propagation of electromagnetic surface waves", J. Phys. D: Appl. Phys. 24, 1025-1048 (1991).

D'autres types de plasma peuvent être utilisés aux mêmes fins. Ainsi, on pourrait employer ceux générés par une décharge de type capacitif, le conduit creux est alors placé entre les deux électrodes planes disposées parallèlement et alimentées par un champ EM. Il est aussi possible d'utiliser un plasma généré par une excitation de type inductif, telle qu'avantageusement produite à l'intérieur du tube creux lorsque ce dernier est placé à l'intérieur de spires, s'étendant de façon coaxiale au tube, alimentées par un champ EM.

### Mise en oeuvre

Afin de ne pas échauffer outre mesure le conduit, ce qui risquerait de l'endommager, une fréquence d'excitation de l'onde préférablement égale ou inférieure à 200 MHz est utilisée.

Ainsi, la densité du plasma créé est limitée. La température du gaz (chauffé par collisions électroniques) est donc réduite.

Une température plus faible peut être obtenue ou une fréquence plus élevée peut néanmoins être employée sans que la température du gaz ne soit trop élevée, à condition cependant d'imposer un régime d'alimentation, par impulsion, de la puissance EM appliquée caractérisé par des temps morts suffisamment longs.

Ce résultat s'obtient en recourant, par exemple, à une modulation en créneau (Figure 4) ou, encore à une modulation de forme sinusoïdale, de la puissance du générateur. La durée du temps mort du créneau est choisie de manière à éviter d'aller au-delà d'une température maximale. On peut aussi combiner l'abaissement de fréquence de l'onde EM et la durée des temps de marche et d'arrêt de l'impulsion de la puissance EM pour ne pas dépasser la température optimale souhaitée.

Le procédé de stérilisation du conduit, dans les exemples présentés, se fait suivant plusieurs étapes, dans un dispositif tel que représenté dans la Figure 1. Un tube creux, par exemple un endoscope, est placé dans la chambre de stérilisation. La source de plasma pour la post-décharge est reliée à la partie supérieure de la chambre de stérilisation et l'excitateur à ondes de surface est reliée à la partie inférieure de la chambre de stérilisation. L'orientation des flèches indique si la vanne contrôlant la circulation des gaz est ouverte (sens du tube) ou fermée (orientation perpendiculaire à l'axe du tube). Dans la première étape représentée sur la Figure 1 (à noter que l'ordre des étapes peut être inversé), on stérilise l'intérieur ainsi que l'extérieur de l'emballage ainsi que le tube creux.

L'extérieur du tube est assurément stérilisé de façon très efficace. Cependant, l'intérieur du tube, compte tenu de la difficulté à faire circuler rapidement un flux de gaz à l'intérieur d'un tube creux de faible diamètre, est stérilisé de façon imparfaite. Dans la deuxième étape de la Figure 1, on stérilise directement l'intérieur de l'endoscope en propageant une onde de surface qui créé le plasma à l'intérieur du tube creux.

Les étapes principales de mise en oeuvre du procédé sont ci-après commentées.

Dans une première étape, comme le montrent les Figures 1A et 2A (première étape), l'extérieur du conduit aussi bien que l'emballage sont soumis à la stérilisation par post-décharge, de préférence selon un des procédés déjà décrits dans la publication de M. Moisan, S. Moreau, M. Tabrizian, J. Pelletier, J. Barbeau et L'H. Yahia intitulée "Procédé de stérilisation d'objets par plasma" ou dans la demande de brevet canadien déposée le 28 mai 1999 et identifiée sous le numéro CA-A-2 273 432, au nom de l'Université de Montréal. Pour ce faire, les espèces actives proviennent d'une source de plasma d'un diamètre typiquement de 25-30 mm. Au cours de cette même étape, les embouts qui sont destinés à s'insérer sur les faces extérieures des extrémités du conduit, sont également stérilisés.

Dans une deuxième étape, c'est l'intérieur du conduit qui est soumis à une stérilisation, cette fois par un plasma d'ondes de surface s'étendant sur toute sa longueur. Pour ce faire, tout d'abord, on utilise préférentiellement un bras articulé muni d'une pince (non représenté sur la Figure 1) afin d'insérer l'extrémité du conduit sur l'embout (diélectrique) du tube (diélectrique) portant l'excitateur d'ondes de surface; l'embout de ce tube s'embranchant sur la face extérieure du conduit; un autre embout du même type relie la sortie du conduit au groupe de pompage. Une fois la stérilisation "intérieure" terminée, le bras articulé (toujours sous ambiance stérile) est utilisé pour dégager le conduit des deux embouts.

Dans une troisième étape, le bras articulé est utilisé pour insérer le conduit dans un sachet d'emballage qui est scellé, par exemple, de façon thermique (thermo-soudure).

Dans une quatrième étape, l'enceinte de stérilisation peut alors être ouverte et le conduit emballé récupéré et transféré dans la pièce où il peut être soit immédiatement utilisé, soit entreposé.

Le procédé décrit ci-haut est donné à titre d'exemple, d'autres variantes qui mettent en oeuvre un plasma d'ondes de surface, sont utilisables pour stériliser l'intérieur du conduit.

Ainsi, parmi les nombreuses variantes possibles, dans la deuxième étape déjà décrite, on peut laisser l'extrémité du conduit, opposée à l'arrivée du gaz, libre. Le pompage et l'évacuation des effluents se fait alors comme dans la première étape (Figure 2B).

Selon une autre variante, le lanceur d'onde est positionné à l'intérieur de l'enceinte et placé à mi-parcours du conduit, assurant ainsi une meilleure uniformité axiale du plasma dans le conduit.

Enfin, selon un autre mode de réalisation il est possible d'inverser l'ordre des deux étapes précédemment décrites, et ce dans la mesure où, il s'avère plus facile, voire même plus rapide, de glisser le conduit dans le lanceur aussi bien que d'insérer les embouts sur le conduit avant même de commencer les opérations de stérilisation, alors que l'enceinte de stérilisation est ouverte à l'air libre.

### Gaz de stérilisation

Un gaz de stérilisation est utilisé pour la production de la post-décharge du plasma et pour la production de l'onde de surface à l'intérieur de l'objet à décontaminer de nature essentiellement diélectrique.

Les deux demandes de brevets EP-A-1181062 et WO-A-2004011039 décrivent des modes particulièrement avantageux de réalisation de la stérilisation par une post-décharge de plasma, un mélange N₂-O₂ (voir aussi M. Moisan, S. Moreau, M. Tabrizian, J. Pelletier, J. Barbeau, L'H. Yahia, "Procédé de stérilisation d'objets par plasma" et demande de brevet canadien, numéro de série du dépôt (28 mai 1999), 2,273,432 au nom de l'Université de Montréal.) ou avec de l'argon pur, ou d'autres gaz rares ou mélanges de gaz comme décrit dans la publication M. Moisan, N. Philip, B. Saoudi intituée "Système et procédé de haute performance pour la stérilisation par plasma gazeux à basse température" et demande dé brevet canadien numéro 2,395,659 déposée le 26 juillet 2002, dans les deux cas grâce aux photons UV.

Une bonne uniformité des espèces actives stérilisantes est obtenue dans l'enceinte de post-décharge de préférence avec un mélange N₂-O₂. À noter cependant que le mélange N₂-O₂ érode davantage les matériaux que l'argon, et ce, à cause de la présence de l'oxygène atomique qui est doté d'une forte réactivité chimique. Ainsi, on peut utiliser l'argon pour stériliser l'intérieur du conduit creux. Le problème de la non uniformité spatiale des espèces actives ne se pose pas. Alors que l'extérieur du conduit peut être avantageusement stérilisé par post-décharge dans le mélange N₂-O₂ approprié. Ainsi, l'érosion de l'intérieur du conduit, la partie la plus délicate de ce dispositif, est minimisée.

Dans l'étape de stérilisation de l'intérieur du conduit par une onde de surface, le débit du gaz de la décharge est ajusté à un niveau :
- suffisamment faible pour ne pas créer de gradient de pression important dans le conduit ; et
- à un niveau suffisamment élevé pour assurer un bon renouvellement du gaz et l'évacuation des effluents de la stérilisation.

En effet, les conditions de stérilisation à partir de gaz rares comme l'argon dépendent de façon critique de la pression locale du gaz car celle-ci agit sur l'intensité d'émission des photons UV comme cela est décrit dans la publication de M. Moisan et de A. Ricard publiée dans Can. J. Physics 55, 1010-1012 (1977). À noter que lorsqu'il existe un gradient de pression important, il faut réajuster la pression ou le débit de gaz en cours de stérilisation afin que chaque section du conduit soit successivement à la pression optimale et reçoive ainsi le flux maximal de photons.

### Lanceurs d'onde de surface

Il existe un nombre important de lanceurs d'onde de surface pouvant être utilisés pour les fins indiquées. Dans le dispositif décrit, un excitateur appelé Ro-box, mentionné dans le brevet américain US-A-4.810.933, a été préférentiellement retenu.

### Exemples de conduits creux

On stérilise de façon particulièrement avantageuse des endoscopes par exemple ceux décrits dans le brevet US-A-6.471.639, des cathéters ou tout ensemble de conduits creux à axes parallèles disposés dans une enveloppe cylindrique ou oblate.

### EXEMPLES

Les exemples ci-après rapportés sont donnés à titre purement illustratif et ne sauraient être interprétés comme constituant une quelconque limitation de l'objet revendiqué.

### Exemple 1 - montage expérimental et résultats correspondants relatif à l'utilisation de la décharge d'onde de surface pour l'inactivation des spores B. subtilis introduits dans un tube creux - Figure 3.

Ce montage montre comment utiliser la propagation d'une onde de surface pour produire un plasma à l'intérieur d'un tube creux en matériau diélectrique sans que celui-ci ne soit endommagé par la chaleur et comment contrôler la valeur de la température du gaz de la décharge gazeuse pour obtenir ce résultat. La température de l'extérieur du tube creux est avantageusement mesurée avec un thermocouple.

Ce mode de réalisation du procédé de l'invention permet de stériliser l'intérieur d'un tube creux. Dans la Figure 5, l'élément (1) représente le tube de décharge , (2) la section de tube de 1 cm de longueur en Téflon^{™} contaminé à l'intérieur, (3) l'excitateur d'onde de surface robox et (4) le plasma.

L'alimentation en puissance de haute fréquence (HF) est constituée d'un amplificateur piloté par un oscillateur, dont la fréquence est fixée à 100 MHz dans l'exemple. L'émission de l'oscillateur est interrompue à intervalle fixe déterminé par ordinateur. Un des modes opératoires choisi étant une impulsion en créneau d'une durée de 10 millisecondes suivi d'un temps mort de 90 millisecondes, donnant lieu à un cadencement de l'impulsion de 10 Hz (Figure 4).

Le fait de ne pas alimenter de façon continue la décharge permet d'ajuster la température du gaz se trouvant dans le tube creux dont on veut stériliser l'intérieur sans endommager le matériau qui le constitue. Plus la durée du temps mort est grande, plus froide sera la décharge dans le tube creux. On notera sur la Figure 4 que le signal de contrôle de l'oscillateur ne va pas exactement à zéro pendant le temps dit mort, ce qui permet de maintenir une décharge minimale (très courte longueur), évitant ainsi d'avoir à relancer l'amorçage de la décharge par une impulsion extérieure.

C'est aussi pour diminuer l'échauffement du gaz que la fréquence de fonctionnement a été abaissée à la plus basse valeur compatible avec le type de boîte d'accord d'impédance utilisé (circuits L (inductance) et C (capacitance) dans l'air), en l'occurrence 100 MHz.

Il apparaît intéressant de modifier le montage pour pouvoir fonctionner encore à plus faible fréquence, par exemple à quelques centaines de kHz, mais il faudra utiliser un système d'accord d'impédance d'un type différent, plus lourd.

La partie principale des endoscopes est faite de Téflon^{™}, qui est un excellent diélectrique mais qui ne supporte pas des températures trop élevées. On considère que la température maximum d'utilisation pour assurer l'intégrité du Téflon^{™} est, en théorie, de 260 degrés Celsius. Mais en pratique, pour éviter toute déformation du Téflon^{™} ainsi que du polymère recouvrant le Téflon^{™} (souvent du polyuréthane), il faut maintenir une température inférieure à 60 degrés Celsius.

### Exemple 2 - Utilisation de deux gaz différents pour effectuer la stérilisation.

A) Dans un premier cas, de l'argon pur a été utilisé pour produire, à l'intérieur du tube creux, une décharge gazeuse d'une puissance HF de 100 MHz, par propagation d'une onde de surface. La décharge a lieu dans un tube en quartz (silice fondue) de 3 mm de diamètre interne (tube dans lequel il a été glissé des sections de 1 cm de longueur de tube en Téflon™, contaminé par *B. subtilis,* voir Figure 5). Pour assurer que la décharge est uniforme le long du tube creux, un très petit débit de gaz (≤ 0.3 cm³/min.) a été utilisé. La pression a été fixée à 40 Pa (0,3 Torr); valeur déterminée à partir des résultats obtenus dans des tubes de 26 mm décrits dans la publication de M. Moisan, N. Philip, B. Saoudi, intitulée "Système et procédé de haute performance pour la stérilisation par plasma gazeux à basse température" et dans la demande de brevet canadien numéro 2,395,659 déposée le 26 juillet 2002, en faisant l'hypothèse que l'intensité de l'émission UV dépend directement de la température électronique, laquelle est fonction d'une loi de similitude en pR (produit pression p par rayon R du tube). L'échauffement du tube alors est faible, moins de 40 à 50 degrés Celsius pour un régime de fonctionnement avec impulsion en créneau uniforme (temps actif=temps mort) à 10 Hz de cadencement. Dans la Figure 3 correspondante, l'élément (1) représente l'oscillateur, (2) l'amplificateur, (3) la ligne bidirectionnelle de mesure de puissance, (4) le bolomètre, (5) la boîte d'accord d'impédance, (6) l'excitateur d'onde de surface Ro-box, (7) la jauge de pression de gaz, (8) le tube de décharge, (9) le générateur de fonction (ordinateur).
   Il a ainsi été observé qu'il était possible de stériliser la section de Téflon™ contaminée en environ 15 minutes. Le niveau maximum de l'intensité UV à la pression utilisée pourrait être ajustée de façon optimale en procédant à une mesure dite d'absorption qui donne la densité de population de l'état d'énergie supérieur de la transition émettant les photons UV.
B) Il a été utilisé un mélange N₂-O₂ dont le pourcentage a été fixé, à l'aide d'un spectromètre optique, de façon à maximiser l'intensité UV émise à 305 nm par observation directe de la lumière de la décharge au moyen d'un spectromètre optique. La décharge a lieu à 100 MHz dans un tube de diamètre interne de 3 mm et la pression fixée autour de 40 Pa (0,3 Torr) le débit étant très faible comme pour l'argon. En régime continu d'alimentation HF, la décharge est trop chaude, et il faut donc passer à un régime de fonctionnement avec temps mort comme décrit dans l'exemple 1 (Figure 4). Ce régime de fonctionnement donne lieu à une température ne dépassant pas 40 Celsius.

### Exemple 3 - Applicateur de champ micro-ondes, de forme linéaire, disposé le long du tube creux à stériliser et extérieurement à celui-ci.

On décrit tout d'abord le schéma de principe du recours à un applicateur linéaire pour stériliser. En faisant référence à la Figure 6, le flux de puissance micro-ondes transmis par le générateur à la ligne de transmission (feeding line) s'écoule par des ouvertures dans la structure de l'applicateur donnant lieu à un champ électrique permettant de créer une décharge dans le tube qui lui fait face, parallèlement. La puissance non utilisée en fin d'applicateur est dissipée dans une charge dite adaptée (afin d'éviter une réflexion de l'onde EM en bout d'applicateur). Il est possible de concevoir un tel système pour que la densité du plasma créé soit uniforme le long du tube à stériliser. L'avantage de ce système est qu'il crée un plasma de bien moindre densité que celui de l'onde de surface. Ainsi, nous pouvons utiliser une alimentation HF fonctionnant à 915 MHz et en régime continu (pas de temps mort) sans échauffer indûment le tube à décharge. L'inconvénient principal semble être la perte de puissance en bout de ligne (prix à payer pour assurer l'uniformité de la décharge le long du tube). L'utilisation décrite ici de l'applicateur linéaire ne concerne que la stérilisation de l'intérieur du tube creux. Pour la stérilisation de l'extérieur de celui-ci, il faut implanter ce système dans l'enceinte de post-décharge décrite dans les Figures 1 et 2.

### Références bibliographiques

[Art] M. Moisan et A. Ricard, Can. J. Physics 55, 1010-1012 (1977).
[Br 1] M. Moisan, S. Moreau, M. Tabrizian, J. Pelletier, J. Barbeau, L'H. Yahia, "Procédé de stérilisation d'objets par plasma" (Demande de brevet canadien, numéro de série du dépôt (28 mai 1999), 2,273,432 au nom de l'Université de Montréal.
M. Moisan, S. Moreau, M. Tabrizian, J. Pelletier, J. Barbeau, L'H. Yahia, "Système et procédé de stérilisation par plasma gazeux à basse température", Demande internationale de brevet (Traité de coopération en matière de brevets (PCT)), numéro PCT/CA00100623 (26 mai 2000) au nom de l'Université de Montréal. Publication de cette demande sous le numéro WO 00/72889 en date du 7 décembre 2000.
[Br 2] M. Moisan, N. Philip, B. Saoudi, "Système et procédé de haute performance pour la stérilisation par plasma gazeux à basse température", demande de brevet canadien numéro 2,395,659 déposée le 26 juillet 2002.
[Br 3] M. Moisan, Z. Zakrzewski, brevet américain 4, 810, 933 (7 mars 1989).
[Sy 1] M. Moisan, Z. Zakrzewski, "Plasma sources based on the propagation of electromagnetic surface waves", J. Phys. D: Appl. Phys. 24 1025-1048 (1991).

## Revendications

1. Procédé de stérilisation d'un objet contaminé de nature diélectrique et comportant au moins une partie creuse, ledit procédé étant **caractérisé en ce que** :
a) on génère un plasma à partir d'un gaz ou d'un mélange de gaz et d'un champ électromagnétique ;
b) on traite l'extérieur dudit objet par le biais d'une post-décharge dudit plasma ; et
c) on traite la partie creuse dudit objet par le biais d'une décharge dudit plasma, ladite décharge étant générée à l'intérieur de ladite partie creuse,
l'étape (c) étant effectuée avant ou après l'étape (b).

2. Procédé selon la revendication 1, dans lequel ledit gaz comprend de l'oxygène moléculaire, de l'azote, du néon, de l'argon, du krypton, du xénon, de l'hélium, de l'oxygène, du monoxyde de carbone, du dioxyde de carbone, des gaz de formule NO_{x,} où x représente 1, 2 ou 3, ou de l'air.

3. Procédé selon la revendication 1, dans lequel ledit mélange comprend au moins deux gaz choisis parmi le groupe constitué par l'oxygène moléculaire, l'azote, le néon, l'argon, le krypton, le xénon, l'hélium, l'oxygène, le monoxyde de carbone, le dioxyde de carbone, les gaz de formule NOₓ où x représente 1,2 ou 3, et l'air.

4. Procédé selon la revendication 1, dans lequel ledit mélange est un mélange N₂-O₂.

5. Procédé de stérilisation d'un objet contaminé de nature diélectrique et comportant au moins une partie creuse, ledit procédé étant **caractérisé en ce que** :
a)on génère un premier plasma à partir d'un gaz ou d'un mélange de gaz et d'un champ électromagnétique ;
b) on traite l'extérieur dudit objet par le biais d'une post-décharge dudit premier plasma;
c) on génère un deuxième plasma à partir d'un gaz ou d'un mélange de gaz et d'un autre champ électromagnétique ;
d) on traite la partie creuse dudit objet par le biais d'une décharge dudit deuxième plasma, ladite décharge étant générée à l'intérieur de ladite partie creuse, l'étape (c) étant effectuée avant ou après l'étape (a) et/ou l'étape (b), et l'étape (d)
étant effectuée après l'étape (c).

6. Procédé selon la revendication 5, dans lequel ledit gaz à l'étape (a) comprend de l'oxygène moléculaire, de l'azote, du néon, de l'argon, du krypton, du xénon, de l'hélium, de l'oxygène, du monoxyde de carbone, du dioxyde de carbone, des gaz de formule NOₓ, où x représente 1, 2 ou 3, ou de l'air.

7. Procédé selon la revendication 5, dans lequel ledit mélange à l'étape (a) comprend au moins deux gaz choisis parmi le groupe constitue par l'oxygène moléculaire, l'azote, le néon, l'argon, le krypton, le xénon, l'hélium, l'oxygène, le monoxyde de carbone, le dioxyde de carbone, les gaz de formule NOₓ, où x représente 1, 2 ou 3, et l'air.

8. Procède selon la revendication 5, dans lequel ledit gaz à l'étape (c) comprend de l'oxygène moléculaire, de l'azote, du néon, de l'argon, du krypton, du xénon, de l'hélium, de l'oxygène, du monoxyde de carbone, du dioxyde de carbone, des gaz de formule NOₓ, où x représente 1, 2 ou 3, ou de l'air.

9. Procédé selon la revendication 5, dans lequel ledit mélange à l'étape (c) comprend au moins deux gaz choisis parmi le groupe constitué par l'oxygène moléculaire, l'azote, le néon, l'argon, le krypton, le xénon, l'hélium, l'oxygène, le monoxyde de carbone, le dioxyde de carbone, les gaz de formule NOₓ, où x représente 1, 2 ou 3, et l'air.

10. Procédé selon la revendication 5, dans lequel le premier plasma est généré à partir d'un mélange N₂-O₂.

11. Procédé selon la revendication 5 ou 10 dans lequel le deuxième plasma est généré à partir d'argon.

12. Procédé selon la revendication 1, dans lequel le champ électromagnétique est celui d'une onde de surface.

13. Procédé selon la revendication 5, dans lequel le champ électromagnétique de l'étape (a) et/ou (c) est celui d'une onde de surface.

14. Procédé selon l'une quelconque des revendications 1 à 13, dans lequel les parois de la ou les partie(s) creuse(s) de l'objet contaminé sont constituées d'un matériau principalement de nature diélectrique sur la plus grande partie de sa longueur, de préférence pour au moins 90 % de sa longueur.

15. Procédé selon l'une quelconque des revendications 1 à 14, dans lequel l'objet contaminé est un conduit creux cylindrique.

16. Procédé selon la revendication 15, dans lequel le rapport obtenu en divisant la longueur du cylindre constitutif de l'objet contaminé par le diamètre du cylindre est compris entre 5.10³ et 0,3.10³.

17. Procédé selon la revendication 15, dans lequel ledit conduit creux comporte au moins deux extrémités libres, chacune des extrémités étant munie d'un embout diélectrique et positionnée dans une enceinte de stérilisation de façon à ce qu'un premier embout soit en contact avec un excitateur d'onde de surface et de façon qu'un deuxième embout soit relié à un système de pompage qui évacue des effluents de la décharge.

18. Procédé selon l'une quelconque des revendications 1 à 14, dans lequel l'objet contaminé est un tube creux.

19. Procédé selon la revendication 12, dans lequel le temps de traitement de la ou des partie(s) creuse(s) par l'onde de surface électromagnétique est compris entre 45 et 120 minutes, de préférence le temps de traitement est de 60 minutes et/ou la température dans le tube creux est comprise entre 30 et 60 degrés Celsius de préférence comprise entre 30 et 45 Celsius.

20. Procédé selon l'une quelconque des revendications 1 à 19, dans lequel la fréquence d'excitation du plasma lors de la décharge est comprise entre 10 kHz et 10 GHz.

21. Procédé selon l'une quelconque des revendications 1 à 19, dans lequel la fréquence d'excitation du plasma lors de la décharge est comprise entre 1 MHz et 2500 MHz.

22. Procédé selon l'une quelconque des revendications 1 à 21, dans lequel un emballage destiné à recevoir ledit objet est traité lors de ladite post-décharge.

23. Procédé selon la revendication 22, dans lequel la partie intérieure de l'emballage est orientée face à une source où est généré ledit plasma de la post-décharge.

24. Procédé selon la revendication 22 ou 23, dans lequel, une fois la stérilisation terminée, l'objet décontaminé est transféré, tout en maintenant une ambiance stérile, dans ledit emballage qui est présent dans une enceinte où est effectué ledit procédé

25. Procédé selon la revendication 24, comprenant l'étape de sceller ledit emballage contenant l'objet décontaminé par le biais d'un processus de thermosoudure.

26. Procédé selon l'une quelconque des revendications 1 à 25, dans lequel l'objet à décontaminer est choisi dans le groupe constitué par les endoscopes, les cathéters et par les ensembles de conduits creux à axes parallèles disposés dans une enveloppe cylindrique ou oblate..

27. Procédé selon l'une quelconque des revendications 1 à 26, dans lequel on traite la partie creuse dudit object et par la suite, on traite l'extérieur dudit objet.

28. Dispositif permettant de stériliser un objet contaminé de nature diélectrique et ayant au moins une partie creuse, ledit dispositif comprenant :
- une enceinte de stérilisation destinée à recevoir ledit objet ;
- une première source de plasma en communication avec ladite enceinte, et destinée à générer un plasma servant à traiter l'extérieur dudit objet par le biais d'une post-décharge ;
- une seconde source .de plasma en communication avec ladite enceinte, et destinée à générer un plasma servant à traiter ladite partie creuse dudit objet par le biais d'une décharge, ladite seconde source comprenant un embout dimensionné de telle sorte que lorsque ce dernier est couplé avec la partie creuse dudit objet, ladite décharge est produite à l'intérieur de la partie creuse ; et
- une sortie en communication avec ladite enceinte et permettant d'évacuer des gaz générés dans l'enceinte.

29. Dispositif selon la revendication 28, comprenant une autre sortie en communication avec l'enceinte, ladite autre sortie comprenant un embout dimensionné de telle sorte que lorsque ce dernier est couplé avec la partie creuse dudit objet, des gaz produits à l'intérieur de la partie creuse, lors de la décharge, sont évacués par le biais de celle-ci de façon à éviter un contact entre ceux-ci et l'extérieur dudit objet.

30. Dispositif selon la revendication 29, **caractérisé en ce que** ledit objet est un tube creux, et **en ce qu'**une des extrémités du tube est couplé avec l'embout de la seconde source de plasma, et que l'autre extrémité du tube creux est couplé avec l'embout de l'autre sortie, de façon à ce que la décharge soit effectuée à l'intérieur du tube évitant ainsi un contact entre l'extérieur du tube et le second plasma, sa décharge et les gaz produits lors de cette dernière.

31. Dispositif selon l'une quelconque des revendications 28 à 30, dans lequel l'enceinte de stérilisation comporte un ou plusieurs supports.

32. Dispositif selon la revendication 31, dans lequel le ou les supports sont réglables quant à leur position dans ladite enceinte.

33. Dispositif selon l'une quelconque des revendications 28 à 32, dans lequel l'enceinte de stérilisation comporte des moyens permettant de manipuler de façon stérile les objets qui y sont placés.

## Claims

1. Process for sterilizing a dielectric contaminated object having at least one hollow part, said process comprising:
a) generating a plasma from a gas or a mixture of gases and an electromagnetic field;
b) treating the exterior of said object by means of an after-glow of said plasma; and
c) treating the hollow part of said object by means of a discharge of said plasma, said discharge being generated inside said hollow part,
step (c) being carried out before or after step (b).

2. Process according to claim 1, wherein said gas comprises molecular oxygen, nitrogen, neon, argon, krypton, xenon, helium, oxygen, carbon monoxide, carbon dioxide, gases of formula NOₓ, in which x represents 1, 2, or 3, or air.

3. Process according to claim 1, wherein said mixture comprises at least two gases selected from the group consisting of molecular oxygen, nitrogen, neon, argon, krypton, xenon, helium, oxygen, carbon monoxide, carbon dioxide, gases of formula NOₓ, in which x represents 1, 2, or 3, and air.

4. Process according to claim 1, wherein said mixture is a N₂-O₂ mixture.

5. Process for sterilizing a dielectric contaminated object having at least one hollow part, said process comprising:
a) generating a first plasma from a gas or a mixture of gases and an electromagnetic field;
b) treating the exterior of said object by means of an after-glow of said first plasma;
c) generating a second plasma from a gas or a mixture of gases and another electromagnetic field; and
d) treating the hollow part of said object by means of a discharge of said second plasma, said discharge being generated inside said hollow part,
step (c) being carried out before or after step (a) and/or step (b), and step (d) being carried out after step (c).

6. Process according to claim 5, wherein said gas in step (a) comprises molecular oxygen, nitrogen, neon, argon, krypton, xenon, helium, oxygen, carbon monoxide, carbon dioxide, gases of formula NOₓ, where x represents 1, 2, or 3, or air.

7. Process according to claim 5, wherein said mixture in step (a) comprises at least two gases selected from the group consisting of molecular oxygen, nitrogen, neon, argon, krypton, xenon, helium, oxygen, carbon monoxide, carbon dioxide, gases of formula NOₓ, where x represents 1, 2, or 3, and air.

8. Process according to claim 5, wherein said gas in step (c) comprises molecular oxygen, nitrogen, neon, argon, krypton, xenon, helium, oxygen, carbon monoxide, carbon dioxide, gases of formula NOₓ, where x represents 1, 2, or 3, or air.

9. Process according to claim 5, wherein said mixture in step (c) comprises at least two gases selected from the group consisting of molecular oxygen, nitrogen, neon, argon, krypton, xenon, helium, oxygen, carbon monoxide, carbon dioxide, gases of formula NOₓ, where x represents 1, 2, or 3, and air.

10. Process according to claim 5, wherein the first plasma is generated from a N₂-O₂ mixture.

11. Process according to claim 5 or 10, wherein the second plasma is generated from argon.

12. Process according to claim 1, wherein the electromagnetic field is one produced by a surface wave.

13. Process according to claim 5, wherein the electromagnetic field of step (a) and/or (c) is one produced by a surface wave.

14. Process according to any one of claims 1 to 13, wherein walls of the hollow part(s) of the contaminated object are made of a material that is mainly of dielectric nature on the most part of its length, preferably on at least 90 % of its length.

15. Process according to any one of claims 1 to 14, wherein the contaminated object is a cylindrical hollow duct.

16. Process according to claim 15, wherein the ratio obtained by dividing the length of the cylinder that constitutes the contaminated object by the diameter of the cylinder is between 5.10³ and 0.3.10³.

17. Process according to claim 15, wherein said hollow duct includes at least two free ends, each end being provided with a dielectric mouthpiece and positioned in a sterilization chamber so that a first mouthpiece is in contact with a surface wave exciter and that a second mouthpiece is connected to a pump system that exhausts effluents from the discharge.

18. Process according to any one of claims 1 to 14, wherein the contaminated object is a hollow tube.

19. Process according to claim 12, wherein treatment of the hollow part(s) with the electromagnetic surface wave lasts between 45 and 120 minutes, preferably 60 minutes and/or the temperature in the hollow tube, during the treatment, is comprised between 30 and 60 degrees Celsius, preferably between 30 and 45 degrees Celsius.

20. Process according to any one of claims 1 to 19, wherein the frequency of excitation of the plasma during the discharge is comprised between 10 kHz and 10 GHz.

21. Process according to any one of claims 1 to 19, wherein the frequency of excitation of the plasma during the discharge is comprised between 1 MHz and 2500 MHz.

22. Process according to any one of claims 1 to 21, wherein a wrapping adapted to receive said object is treated during said after-glow.

23. Process according to claim 22, wherein the interior part of the wrapping is oriented to face a source where said after-glow plasma is produced.

24. Process according to claim 22 or 23, wherein, once sterilization is over, the sterilized object is transferred, while keeping a sterile environment, into said wrapping that is present in a chamber where said process is carried out.

25. Process according to claim 24, comprising the step of sealing said wrapping containing the sterilized object by means of a thermo-welding process.

26. Process according to any one of claims 1 to 25, wherein the object to sterilize is selected from the group consisting of endoscopes, catheters and hollow ducts assemblies with parallel axes disposed in a cylindrical or oblong envelope.

27. Process according to any one of claims 1 to 26, wherein the hollow part of said object is treated, and then, the exterior of said object is treated.

28. Device for sterilizing a dielectric contaminated object and having at least one hollow part, said device comprising:
- a sterilization chamber adapted to receive said object;
- a first plasma source in communication with said chamber, and adapted to generate a plasma to be used for treating the exterior of said object by means of an after-glow;
- a second plasma source in communication with said chamber, and adapted to generate a plasma to be used for treating said hollow part of said object by means of a discharge, said second source comprising a mouthpiece dimensioned so that when the latter is coupled with the hollow part of said object, said discharge is produced inside the hollow part; and
- an outlet in communication with said chamber and allowing to exhaust gases produced in said chamber.

29. Device according to claim 28, comprising another outlet in communication with said chamber, said another outlet comprising a mouthpiece dimensioned so that when the latter is coupled with the hollow part of said object, gases produced inside the hollow part, during the discharge, are exhausted through the latter so as to avoid contact of the gases with the exterior of said object.

30. Device according to claim 29, **characterized in that** said object is a hollow tube, and **in that** one of the ends of the tube is coupled with the mouthpiece of the second plasma source, and that the other end of the hollow tube is coupled with the mouthpiece of the other outlet, so that the discharge is carried out inside the tube, thereby avoiding contact between the exterior of the tube and the second plasma, its discharge and the gases produced during the latter.

31. Device according to any one of claims 28 to 30, wherein the sterilization chamber includes one or more supports.

32. Device according to claim 31, wherein the support(s) are adjustable with respect to their position in said chamber.

33. Device according to any one of claims 28 to 32, wherein said sterilization chamber includes means for handling said object placed therein in a sterile manner.

## Patentansprüche

1. Sterilisationsverfahren für ein verunreinigtes Objekt, das dielektrisch ist und mindestens einen Hohlraum aufweist, wobei das Verfahren **dadurch gekennzeichnet ist, daß**:
a) ein Plasma erzeugt wird aus einem Gas oder einem Gasgemisch und einem elektromagnetischen Feld;
b) das Äußere des Objekts mittels einer Nachentladung des Plasmas behandelt wird; und
c) der Hohlraum des Objekts mit einer Entladung des Plasmas behandelt wird, wobei die Entladung im Inneren des Hohlraums erzeugt wird,
wobei der Schritt (c) vor dem Schritt (b) ausgeführt wird.

2. Verfahren nach Anspruch 1, wobei das Gas molekularen Sauerstoff, Stickstoff, Neon, Argon, Krypton, Xenon, Helium, Sauerstoff, Kohlenmonoxid, Kohlendioxid, Gase der Formel NOₓ, wobei x 1, 2 oder 3 darstellt, oder Luft umfaßt.

3. Verfahren nach Anspruch 1, wobei das Gemisch mindestens zwei Gase umfaßt, die aus der Gruppe gewählt werden, die aus molekularem Sauerstoff, Stickstoff, Neon, Argon, Krypton, Xenon, Helium, Sauerstoff, Kohlenmonoxid, Kohlendioxid, Gasen der Formel NOₓ, wobei x 1, 2 oder 3 darstellt, und Luft besteht.

4. Verfahren nach Anspruch 1, wobei das Gemisch ein Gemisch N₂-O₂ ist.

5. Sterilisationsverfahren für ein verunreinigtes Objekt, das dielektrisch ist und mindestens einen Hohlraum aufweist, wobei das Verfahren **dadurch gekennzeichnet ist daß**:
a) ein Plasma erzeugt wird aus einem Gas oder einem Gasgemisch und einem elektromagnetischen Feld;
b) das Äußere des Objekts mit einer Nachentladung des Plasmas behandelt wird;
c) ein zweites Plasma aus einem Gas oder einem Gasgemisch und einem anderen elektromagnetischen Feld erzeugt wird;
d) der Hohlraum des Objekts mittels einer Entladung des zweiten Plasmas behandelt wird, wobei die Entladung im Inneren des Hohlraums erzeugt wird,
wobei der Schritt (c) vor oder nach dem Schritt (a) und/oder dem Schritt (b) ausgeführt wird, und der Schritt (d) nach dem Schritt (c) ausgeführt wird.

6. Verfahren nach Anspruch 5, wobei das Gas des Schrittes (a) molekularen Sauerstoff, Stickstoff, Neon, Argon, Krypton, Xenon, Helium, Sauerstoff, Kohlenmonoxid, Kohlendioxid, Gase der Formel NOₓ, wobei x 1, 2 oder 3 darstellt, oder Luft umfaßt.

7. Verfahren nach Anspruch 5, wobei das Gemisch des Schrittes (a) mindestens zwei Gase umfaßt, die aus der Gruppe gewählt werden, die aus molekularem Sauerstoff, Stickstoff, Neon, Argon, Krypton, Xenon, Helium, Sauerstoff, Kohlenmonoxid, Kohlendioxid, Gasen der Formel NOₓ, wobei x 1, 2 oder 3 darstellt, und Luft besteht.

8. Verfahren nach Anspruch 5, wobei das Gas des Schrittes (c) molekularen Sauerstoff, Stickstoff, Neon, Argon, Krypton, Xenon, Helium, Sauerstoff, Kohlenmonoxid, Kohlendioxid, Gase der Formel NOₓ, wobei x 1, 2 oder 3 darstellt, oder Luft umfaßt.

9. Verfahren nach Anspruch 5, wobei das Gemisch des Schrittes (c) mindestens zwei Gase umfaßt, die aus der Gruppe gewählt werden, die aus molekularem Sauerstoff, Stickstoff, Neon, Argon, Krypton, Xenon, Helium, Sauerstoff, Kohlenmonoxid, Kohlendioxid, Gasen der Formel NOₓ, wobei x 1, 2 oder 3 darstellt, und Luft besteht.

10. Verfahren nach Anspruch 5, wobei das erste Plasma aus einem Gemisch N₂-O₂ erzeugt wird.

11. Verfahren nach Anspruch 5 oder 10, wobei das zweite Plasma aus Argon erzeugt wird.

12. Verfahren nach Anspruch 1, wobei das elektromagnetische Feld das Feld einer Oberflächenwelle ist.

13. Verfahren nach Anspruch 5, wobei das elektromagnetische Feld des Schrittes (a) und/oder (b) das Feld einer Oberflächenwelle ist.

14. Verfahren nach irgendeinem der Ansprüche 1 bis 13, wobei die Wände des Hohlraums oder der Hohlräume des verunreinigten Objekts über den größten Teil seiner Länge, vorzugsweise mindestens 90% seiner Länge, aus einem im wesentlichen dielektrischen Material bestehen.

15. Verfahren nach irgendeinem der Ansprüche 1 bis 14, wobei das verunreinigte Objekt eine hohle, zylinderförmige Leitung ist.

16. Verfahren nach Anspruch 15, wobei das Verhältnis, das man erhält, indem man die Länge des Zylinders, der das verunreinigte Objekt darstellt, durch den Durchmesser des Zylinders teilt, zwischen 5·10³ und 0,3·10³ liegt.

17. Verfahren nach Anspruch 15, wobei die hohle Leitung mindestens zwei freie Enden aufweist, wobei jedes der Enden mit einem dielektrischen Endstück versehen wird, und in einem Sterilisationsbehälter positioniert wird, so, daß ein erstes Endstück in Kontakt steht mit einem Oberflächenwellen-Erreger und so, daß ein zweites Endstück mit einem Pumpsystem in Verbindung steht, das die Abgase der Entladung absaugt.

18. Verfahren nach irgendeinem der Ansprüche 1 bis 14, wobei das verunreinigte Objekt ein hohles Rohr ist.

19. Verfahren nach Anspruch 2, wobei die Behandlungsdauer des Hohlraums oder der Hohlräume durch die elektromagnetische Oberflächenwelle zwischen 45 und 120 Minuten liegt, vorzugsweise ist die Behandlungsdauer 60 Minuten, und/oder die Temperatur in dem hohlen Rohr liegt zwischen 30 und 60 Grad Celsius, vorzugsweise zwischen 30 und 45 Grad Celsius.

20. Verfahren nach irgendeinem der Ansprüche 1 bis 19, wobei die Erregungsfrequenz des Plasmas bei der Entladung zwischen 10 kHz und 10 GHz liegt.

21. Verfahren nach irgendeinem der Ansprüche 1 bis 19, wobei die Erregungsfrequenz des Plasmas bei der Entladung zwischen 1 MHz und 2500 MHz liegt.

22. Verfahren nach irgendeinem der Ansprüche 1 bis 21, wobei eine Verpackung, die das Objekt aufnehmen soll, bei der Nachentladung behandelt wird.

23. Verfahren nach Anspruch 22, wobei der Innenraum der Verpackung zu einer Quelle hin ausgerichtet wird, wo das Plasma der Nachentladung erzeugt wird.

24. Verfahren nach Anspruch 2 oder 3, wobei nach Beendigung der Sterilisation das dekontaminierte Objekt unter Aufrechterhaltung der sterilen Umgebung in die Verpackung transferiert wird, die in einem Behälter anwesend ist, in dem das Verfahren durchgeführt wird.

25. Verfahren nach Anspruch 24, das das Versiegeln der Verpackung, die das dekontaminierte Objekt enthält, mit Hilfe eines thermischen Schweißverfahrens umfaßt.

26. Verfahren nach irgendeinem der Ansprüche 1 bis 25, bei dem das zu reinigende Objekt gewählt wird in der Gruppe, die aus Endoskopen, Kathetern und Ensembles von hohlen Leitungen mit parallelen Achsen, die in einer zylindrischen oder oblaten Hülle angeordnet sind besteht.

27. Verfahren nach irgendeinem der Ansprüche 1 bis 26, wobei der Hohlraum des Objekts behandelt wird, und danach das Äußere des Objekts behandelt wird.

28. Anordnung, die es erlaubt, ein verunreinigtes Objekt zu sterilisieren, das dielektrisch ist und mindestens einen Hohlraum aufweist, wobei die Anordnung folgendes umfaßt:
- einen Sterilisationsbehälter, der zum Aufnehmen des Objektes dient;
- eine erste Plasmaquelle, die mit dem Behälter in Verbindung steht, um ein Plasma zu erzeugen, das dazu dient, das Äußere des Objekts mit einer Nachentladung zu reinigen;
- eine zweite Plasmaquelle, die mit dem Behälter in Verbindung steht, um ein Plasma zu erzeugen, das dazu dient, den Hohlraum des Objekts mit einer Entladung zu reinigen, wobei die zweite Quelle ein Endstück umfaßt, dessen Abmessungen so beschaffen sind daß, wenn letzteres mit dem Hohlraum des Objekts gekoppelt ist, die Entladung im Inneren des Hohlraums erzeugt wird; und
- einen Ausgang, der mit dem Behälter in Verbindung steht und es erlaubt, die in dem Behälter erzeugten Gase abzusaugen.

29. Anordnung nach Anspruch 28, die einen anderen Ausgang umfaßt, der mit dem Behälter in Verbindung steht, wobei dieser andere Ausgang ein Endstück umfaßt, das so bemessen ist, daß, wenn letzteres mit dem Hohlraum des Objekts gekoppelt ist, die bei der Entladung im Inneren des Hohlraums entstehenden Gase durch dieses abgesaugt werden, so daß ein Kontakt zwischen ihnen und dem Äußeren des Objekts vermieden wird.

30. Anordnung nach Anspruch 29, **dadurch gekennzeichnet, daß** das Objekt ein hohles Rohr ist, und dadurch, daß ein Ende des Rohrs mit dem Endstück der zweiten Plasmaquelle gekoppelt ist und daß das andere Ende des hohlen Rohrs mit dem Endstück des anderen Ausgangs gekoppelt ist, so daß die Entladung im Inneren des Rohrs durchgeführt wird, so daß ein Kontakt zwischen dem Äußeren des Rohrs und dem zweiten Plasma, seiner Entladung und den bei letzterer erzeugten Gasen vermieden wird.

31. Anordnung nach irgendeinem der Ansprüche 28 bis 30, wobei der Sterilisationsbehälter einen oder mehrere Stützen umfaßt.

32. Anordnung nach Anspruch 31, wobei die Stützen in Bezug auf ihre Position in dem Behälter einstellbar sind.

33. Anordnung nach irgendeinem der Ansprüche 28 bis 32, wobei der Sterilisationsbehälter Mittel umfaßt, die es erlauben, die darin plazierten Objekte steril zu manipulieren.
